# EUROPEAN PATENT APPLICATION

(11) **EP 1 543 845 A2**
(43) Date of publication of application: **22.06.2005**
(21) Application number: 04251199.8
(22) Date of filing: 02.03.2004
(51) Int. Cl.: A61L 15/28, A61L 15/34, A61L 15/42

(54) **Densification agent and oil treated cellulose fibers**

(30) Priority: 19.12.2003 US 741231
(71) Applicant: Weyerhaeuser Company, Federal Way, WA 98063-9777 (US)
(72) Inventor: Hamilton, Robert T., Seattle, WA 98107 (US); West, Hugh, Seattle, WA 98115 (US)
(74) Representative: Hayes, Adrian Chetwynd

(57) **Abstract**

Densification agent and oil treated cellulose fibers exhibit densification properties that are superior to oil treated fibers that have not been treated with a densification agent and cellulose fibers that have not been treated with oil or a densification agent. The densification agent and oil treated cellulose fibers are useful in absorbent articles that may contain superabsorbent materials.

## Description

### FIELD OF THE INVENTION

The present invention relates to cellulose fibers that have been treated with an oil and a densification agent to modify the properties of the cellulose fibers and to methods for producing such modified cellulose fibers.

### BACKGROUND OF THE INVENTION

In the production of absorbent articles, such as diapers and incontinent devices, it is known to combine superabsorbent materials and cellulose fibers to form an absorbent core. The absorbent core receives fluid to be absorbed and retains the fluid. When superabsorbent materials are in the form of powder or small particles, it is a challenge to retain the superabsorbent material in the absorbent core which comprises a matrix of fibers, commonly cellulose fibers. Various methods of retaining superabsorbent material in an absorbent core have been described. For example, it has been proposed that the cellulose fibers be embedded within the surface of the superabsorbent material.

Another approach described in International Publication Nos. WO 94/04352 and WO 94/04351 assigned to the assignee of the present application provides polymeric or non-polymeric binders between the superabsorbent material and the fiber. The binder is described as binding the superabsorbent material to the fiber through hydrogen or coordinate covalent bonds. The noted international publications describe that the addition of small amounts of moisture to the particles or fibers is desirable to promote binding between the superabsorbent material and the fibers. The moisture is described as being provided naturally by the ambient environment such as when the relative humidity of the environment where the superabsorbent material, binder and fibers are combined is approximately 60% to 75%, or higher. In instances where it is determined that the moisture is necessary and the relative humidity is below a desired level, capital equipment can be used in order to humidify the manufacturing location where the superabsorbent material, binder and fibers are contacted. While humidifying the manufacturing location is effective, it requires a capital investment and increases the costs of production.

Retaining the superabsorbent material in an absorbent core over an extended period of time is another challenge that faces manufacturers. The retention of superabsorbent materials in the absorbent core may fail over time for a number of reasons such as vigorous handling of the absorbent core which results in dislodgment of the superabsorbent material or a reduction in the moisture content of the absorbent core.

Diapers including an absorbent core of superabsorbent material and cellulose fibers are typically manufactured by a process that combines cellulose fibers and superabsorbent material. In such a process, rolls or bales of cellulose fibers without superabsorbent material are fiberized by a fiberizing apparatus such as a hammermill. These fiberized cellulose fibers are entrained in air and superabsorbent material is introduced to the air entrained fibers. The air entrained combination of cellulose fibers and superabsorbent material is delivered to an air lay device such as a pad former, which draws the fibers and superabsorbent material onto a screen and forms the fibers and superabsorbent material into a particular shape. These formed pads are then removed from the pad former for further processing, including subjecting the formed pads to compression in order to densify the pad by decreasing its thickness.

Reducing the thickness of the formed pads which are used in diapers is important to diaper manufacturers so that they can reduce the size of packaging which allows them to ship more diapers per volume and to display a larger number of diapers in a limited amount of shelf space. In addition, consumers find thinner diapers more desirable.

The inventors of the subject application have described in their prior Application Serial No. 10/635,062, filed on August 5, 2003, the use of oil applied to fibers or superabsorbent material to achieve attachment of superabsorbent material to fibers. The inventors' additional work in this area has led them to observe that when oil is applied to fibers and pads are formed from such oil treated fibers, the pads when compressed to increase their density do not densify to as great a degree as pads that include fibers that have not been treated with an oil.

With this background, the present inventors have worked to address the challenges above and have developed compositions and methods that employ oil to assist in the retention of superabsorbent materials and which can be compressed to achieve articles of desirable densities.

### SUMMARY OF THE INVENTION

The present invention provides fibers treated with oil and a densification agent that are useful in absorbent cores formed from the treated fibers and superabsorbent materials. The compositions of the present invention can be formed into absorbent articles for absorbing fluids such as aqueous fluids like urine or blood. The compositions are useful in methods for retaining superabsorbent materials in webs or masses of fibers commonly used as absorbent structures in absorbent articles such as diapers, incontinent devices and feminine hygiene products. The methods provide absorbent structures that are able to retain superabsorbent materials at a level that manufacturers of absorbent articles should find desirable. The compositions of the present invention can be compressed to densities that manufacturers of absorbent articles should find desirable.

In one aspect, the present invention relates to a cellulose pulp sheet that includes cellulose fibers, an oil applied to the cellulose fibers, and a densification agent applied to the cellulose fibers. The cellulose pulp sheet can be fiberized into individualized fibers, laid into a pad, and then compressed. The invention also relates to a method for producing a cellulose pulp sheet which includes the steps of providing a cellulose pulp sheet, applying an oil to the cellulose pulp sheet, and applying a densification agent to the cellulose pulp sheet.

In another aspect, the present invention relates to a method for producing a densified web of cellulose fibers that includes the step of providing cellulose fibers. The cellulose fibers are treated with an oil and a densification agent before being fiberized. The fiberized cellulose fibers are compressed to form a densified web.

In yet another aspect, the present invention relates to a method for modifying the densification properties of cellulose fibers treated with an oil. In this aspect, the fibers before treatment with the oil, and after application and removal of a compression load, can be densified to a first density. The oil treated fibers when subjected to the same compression load and release can be densified to a second density wherein the first density is greater than the second density. The method of this aspect of the present invention includes applying a densification agent to the oil treated fibers. The densification agent is applied to the oil treated fibers in an amount that results in the fibers treated with the oil and the densification agent densifying to a third density when subjected to and released from the compression load. The third density being greater than the first density.

Manufacturers of absorbent articles will find the oil and densification agent treated fibers of the present invention useful in their absorbent products due to the densification properties of cellulose pulp fibers treated in accordance with the present invention. The methods of the present invention provide suitable means for producing the cellulose pulp fibers that exhibit superabsorbent retention properties and densification properties that absorbent article manufacturers should find desirable.

### BRIEF DESCRIPTION OF THE DRAWINGS

The foregoing aspects and many of the attendant advantages of this invention will become more readily appreciated as the same become better understood by reference to the following detailed description, when taken in conjunction with the accompanying drawings, wherein:
FIGURE 1 is a graph illustrating the results of compression testing to determine the densification properties of absorbent structures containing cellulose fibers treated in accordance with the present invention;
FIGURE 2 is a graph illustrating the results of compression testing to determine the densification properties of structures containing cellulose fibers treated in accordance with the present invention and superabsorbent materials; and
FIGURE 3 is a schematic illustration of a wet laid web manufacturing line illustrating the application of a densification agent to a wet laid web of cellulose fibers in accordance with the present invention.

### DETAILED DESCRIPTION OF THE PREFERRED EMBODIMENT

As used herein, the term "fiber" refers to natural or synthetic fibers. Such fibers may be physically pretreated, e.g., by subjecting the fibers to steam, or chemically treated, e.g., by crosslinking the fibers. The fibers may also be twisted or crimped as desired.

A particular type of fiber are cellulose fibers. A particular example of a cellulose fiber is wood pulp fiber. Wood pulp fibers can be hardwood pulp fibers or softwood pulp fibers. The pulp fibers may be chemical, thermomechanical, chemithermomechanical or combinations thereof. Such wood pulp fibers can be obtained from well known chemical processes such as the kraft or sulfite processes. Other cellulose fibers include lyocell, linen, chopped silk fibers, bagasse, hemp, jute, rice, wheat, bamboo, corn, sisal, cotton, flax, kenaf, peat moss, and mixtures thereof. When the fibers are cellulose fibers, they may be pretreated with chemicals to result in lignin or cellulose-rich fiber surfaces. In addition, the fibers may be bleached.

Examples of synthetic fibers include acrylic, polyester, carboxylated polyolefin, and polyamine fibers.

As used herein, the term "superabsorbent material" refers to polymers that swell on exposure to water and form a hydrated gel (hydrogel) by absorbing large amounts of water. Superabsorbent materials exhibit the ability to absorb large quantities of liquid, i.e., in excess of 10 to 15 parts of liquid per part thereof. These superabsorbent materials generally fall into three classes, namely starch graft copolymers, crosslinked carboxymethylcellulose derivatives and modified hydrophilic polyacrylates. Examples of such absorbent polymers are hydrolyzed starch-acrylonitrile graft copolymer, a neutralized starch-acrylic acid graft copolymer, a saponified acrylic acid ester-vinyl acetate copolymer, a hydrolyzed acrylonitrile copolymer or acrylamide copolymer, a modified crosslinked polyvinyl alcohol, a neutralized self-crosslinking polyacrylic acid, a crosslinked polyacrylate salt, carboxylated cellulose, and a neutralized crosslinked isobutylene-maleic anhydride copolymer.

Superabsorbent particles are available commercially, for example starch graft polyacrylate hydrogel fines (IM 1000F) from Hoechst-Celanese of Portsmouth, VA, or larger particles such as granules. Other superabsorbent particles are marketed under the trademarks SANWET (supplied by Sanyo Kasei Kogyo Kabushiki Kaisha), SUMIKA GEL (supplied by Sumitomo Kagaku Kabushiki Kaisha and which is emulsion polymerized and spherical as opposed to solution polymerized ground particles), FAVOR (supplied by Stockhausen of Greensboro, North Carolina), and NORSOCRYL (supplied by Atochem).

The term oil as used generally applies to a wide range of substances. Oils may be derived from animals or from plant seeds or nuts, and these types of oils tend to be chemically identical with fats, with the only difference being one of consistency at room temperature. Animal and plant oils are composed largely of triglycerides of the fatty acids, oleic, palmitic, stearic, and linolenic acid. Oils may also be derived from petroleum sources. Petroleum-based oils generally include a mixture of hydrocarbons. As used herein, the term "oil" refers to oils that have melting points below the temperature at which the oil is applied to the fibers as described below in more detail: Such temperature will generally be below 25°C, but could be higher. If the melting point of the oil is greater than the ambient temperature at which the oil is applied to the fibers, the oil can be heated to liquefy it. This ensures that the oils remain liquid during their application to the fibers. Oils useful in the present invention should also have a vapor pressure sufficiently low to prevent evaporation either during their application or during use.

The oil should not penetrate the walls of the fibers so rapidly that it becomes unavailable to retain the superabsorbent material when superabsorbent material is contacted with the oil treated fibers. The oil preferably resides on the surface of the fibers during the useful life of the absorbent article made from the fibers. To that end, oils of higher molecular weight penetrate the fiber wall more slowly than oils of a lower molecular weight.

Examples of "oils" as that term is used herein include fats and their component fatty acids. As described above, fats are naturally occurring esters of long chain carboxylic acids and the triol glycerol. These esters are also referred to as triglycerides. The hydrolysis of fats yields glycerol and three component carboxylic acids. These straight chain carboxylic acids which may be obtained from the hydrolysis of fats are called fatty acids and include one carboxylic acid group. Fatty acids may be saturated or unsaturated. The most common saturated fatty acids are lauric acid, myristic acid, palmitic acid, and stearic acid. Other fatty acids include oleic acid, linoleic acid, and linolenic acid. Generally, the melting point of a fat depends on the amount of unsaturation in the fatty acids. Fats with a preponderance of unsaturated fatty acids generally have melting points below about 25°C. Specific examples of oils as that term is used herein include soybean oil, cottonseed oil, linseed oil, tung oil, castor oil, coconut oil, olive oil, canola oil, safflower oil, com oil or jojoba oil. Jojoba oil is a light yellow liquid at room temperature that is not technically an oil or fat, but rather is a wax. A wax is an ester of fatty acids with long chain monohydric alcohols. The term oil as used herein is intended to include jojoba oil and other waxes that are liquid at temperatures that they are applied to fibers. It should be understood that the foregoing is a list of exemplary oils and that oils useful in the context of the present invention are not necessarily limited to the foregoing oils. It should be understood that use of the term "oil" in this application refers not only to the oil itself comprising a mixture of various fat and fatty acid components, but also includes the individual isolated fats, and the isolated fatty acids that result when the fats are hydrolyzed. For example, the term "oil" as used herein also refers to the fatty acids oleic, palmitic, stearic, and linolenic, that form the most common triglycerides in many oils derived from animals and plants and would be useful to retain superabsorbent material in an absorbent structure comprising oil-treated fibers and superabsorbent material.

The term "oil" as used herein also refers to unsubstituted alkanes, alkenes, alkynes, cycloalkanes, cycloalkenes, cycloalkynes, aromatics, and mixtures thereof derived from petroleum or animal sources that have melting points below the temperature at which the oil is applied to the fibers, e.g., about 25°C. Such oils are generally derived from petroleum sources, but may also be derived from animal sources. Oils of this type useful in the present invention should have vapor pressure sufficiently low to prevent evaporation of the oil during application or use. Specific examples of these types of oils include mineral oil, paraffin oil, hexadecane, squalane, and squalene.

As used herein, mineral oil is an example of a highly refined liquid petroleum derivative. Mineral oil is light, clear, colorless, and odorless and is also referred to as medicinal oil. Mineral oil is used medicinally as an internal lubricant and for the manufacture of salves and ointments.

Paraffin oil is an example of an oil that is either pressed or dry distilled from paraffin distillate obtained from the distillation of petroleum.

Squalane is an example of an alkane derived from animal sources, such as the sebum. Squalene is an example of an alkene; more specifically, a terpene derived from animal sources, such as the human sebum or shark liver oil. Squalene may also be isolated from oils derived from plants, such as olive oil, wheat germ oil, rice bran oil, and yeast.

As used herein, the term saccharide refers to mono-, di-, oligo-, and polysaccharides.

Monosaccharides are carbohydrates that cannot be hydrolyzed into smaller, simpler carbohydrates. Examples of monosaccharides include glucose, fructose, glyceraldehyde, dihydroxyacetone, erythrose, threose, ribose, deoxyribose, galactose, and the like.

Disaccharides are carbohydrates that on a molar basis undergo hydrolysis to produce only two moles of a monosaccharide. Examples of disaccharides include maltose, sucrose, cellobiose, lactose, and the like.

Oligosaccharides are carbohydrates that on a molar basis undergo hydrolysis to produce 3 to 10 moles of a monosaccharide. Examples of oligosaccharides include those found in corn syrups and other mixtures of breakdown products from polysaccharides, and the like.

Polysaccharides are carbohydrates that on a molar basis undergo hydrolysis to produce more than ten moles of a monosaccharide. Examples of polysaccharides include starch, chitin, hemicelluloses such as galactomannan, other polysaccharides found in seaweed, and the like.

Active(s) as that term is used herein refers to the non-water components of a composition. For instance, for high fructose corn syrup the term "actives" refers to the solids content of the high fructose corn syrup.

It should be understood that the term saccharide as used herein not only refers to individual saccharides such as glucose, fructose, or lactose, but also includes mixtures of monosaccharides, disaccharides, oligosaccharides, and/or polysaccharides.

Examples of saccharides that include a mixture of monosaccharides, disaccharides, oligosaccharides, or polysaccharides include corn syrup, high fructose corn syrup, and honey. Corn syrup is generally a mixture of dextrose (glucose), maltose, and maltodextrines and is available from numerous commercial sources. High fructose corn syrup generally includes fructose, dextrose, disaccharides, and other saccharides. Corn syrup and high fructose corn syrup typically are available as aqueous solutions and have a solids content that ranges from 70 to 85 wt. %. An exemplary high fructose corn syrup is available from Archer-Daniels Midland Company under the trademark Corn Sweet® 42. It should be understood that other high fructose corn syrups that are available from Archer-Daniels Midland Company and other commercial sources are useful in the present invention.

Honey useful in accordance with the present invention contains fructose and glucose as the predominate carbohydrates, with- maltose and sucrose present in small percentages. Honey is available from numerous commercial sources.

In accordance with the present invention, the oil can be applied to the fiber in a number of different ways. The particular way that oil is applied to the fibers is not critical. Examples of techniques for applying oil to the fibers include the use of a gravure-type roll coater to coat a web of the fibers. Alternatively, oil can be sprayed onto a web of the fibers or the fibers can be immersed in a bath of oil. The oil may also be added to the fibers as a web of the fibers is being broken up, such as in a hammermill. The amount of oil applied to the fibers should be sufficient to achieve retention of superabsorbent material, but not so much as to have a significant adverse affect on the fluid absorption properties of the fibers, such as the fluid acquisition rate or the amount of fluid absorbed by a web of the fibers. Manufacturers of absorbent articles that include absorbent structures containing oil-treated fibers desire that the fluid absorption properties of such structures be similar to or superior to the fluid absorption properties of the absorbent structures that the manufacturer is considering replacing. Ideally, the absorbent structures would exhibit fluid acquisition properties that are at least as desirable as the fluid acquisition properties of similar absorbent structures manufactured from untreated fibers. The amount of oil applied to the fibers should also not be so great that it adversely impacts the fiberization of the web of oil-treated fibers. Suitable amounts of oil applied to the fibers include about 0.5 wt. % to about 20 wt. % oil based on the weight of oven dried fibers. A narrower range is 1.0 wt. % to about 15 wt. % oil based on the weight of oven dried fibers and an even narrower range is 1.0 wt. % to about 10 wt. % oil based on the weight of oven dried fibers.

The form of the fibers to which the oil is applied can vary. If a roll coater is used, the fibers can be in the form of a sheet of fibers. For example, the oil can be applied to a wet laid sheet of fibers having a basis weight of at least 350 grams per meter² and a density of at least about 400 kg/meter³.

The oil may be added neat, or it may be diluted with solvent that evaporates after application of the oil to the fibers. The solvent should not adversely affect the attachment of superabsorbent material to the fibers or the fluid acquisition and fluid retention properties of an absorbent article that contains the treated fibers.

In accordance with the present invention, a densification agent is applied to the oil treated fibers in order to modify the densification properties of the oil treated fibers. As discussed above, and as illustrated in the examples in this application, fibers treated with oils as described above, when subjected to and released from a compression load, do not densify to as high a density as fibers that have not been treated with an oil when they are subjected to the same compression load.

When the oil treated fibers are treated with densification agent in accordance with the present invention, the oil and densification agent treated fibers when subjected to and released from a compression load densify to a density that is higher than the density that is achieved when fibers that have not been treated with oil and a densification agent are subjected to the same compression loading and releasing.

Densification agents useful in accordance with the present invention include saccharides, agents having at least one hydrogen bonding functionality and saccharides in combination with at least one agent having hydrogen bonding functionality. Useful saccharides have been described above. The solids content of the saccharide applied to the cellulose pulp sheet as a densification agent is preferably less than about 65 wt. %. When corn syrup, high fructose corn syrup, honey, or sucrose is used as the source of saccharide, they can be diluted with water in order to reduce the solids content to below about 65 wt. %. The following description of the application of the densification agent proceeds with reference to a cellulose pulp sheet. It should be understood that the densification agents can be applied to fibers that are in forms other than a cellulose pulp sheet, for example, fibers that have been separated from a pulp sheet.

In accordance with the present invention, the amount of densification agent added to the cellulose pulp sheet can vary over a wide range. Lower amounts of densification agent actives in the treated cellulose sheets are within the present invention; for example, amounts down to about 0.5 wt. % based on the dry fiber content of the cellulose pulp sheet are within the scope of the present invention. On the upper end, the amount of densification agent added to the cellulose pulp sheet is generally limited to an amount that maintains the water content of the cellulose pulp sheet below about 20 wt. %. In preferred embodiments of the present invention, the densification agent is added to the cellulose pulp sheet in an amount that results in an actives content of less than about 20 wt. % based on the weight of dry cellulose fiber in the treated cellulose pulp sheet and more preferably less than about 10 wt. %. Useful results are achieved with actives loading ranging from about 3 to about 8 wt. % based on dry fiber. Sufficient amounts of densification agent actives should be added to the cellulose pulp sheet so that when the cellulose pulp sheet is fiberized the resulting fibers exhibit densification properties that are superior to the densification properties of fibers that have been treated with an oil, but not been treated with a densification agent. As noted above, such actives content can be achieved using a densification agent that has been diluted with water. The extent of the dilution of the densification agent, and therefore the ratio of water to densification agent actives, should be selected so that the desired degree of densification agent actives can be achieved without introducing so much water to the pulp sheet that the pulp sheet becomes difficult to fiberize due to the addition of excess water. It is well known that pulp which is too wet is difficult to fiberize. The dilution should not be so great that when the densification agent solution is added to the cellulose pulp sheet to achieve the desired densification agent active content, the densification agent and oil treated cellulose pulp sheet does not exhibit the desired densification properties. A water content of the cellulose pulp sheet after being treated with densification agent of about 10 wt. % based on the weight of the total product is exemplary. Lower water contents are contemplated with an upper limit of about 15-20 wt. % based on the need to avoid poor fiberization.

The densification agent is applied to the cellulose pulp sheet in a number of different ways. The present invention is not limited to any particular application technique. Examples of suitable application techniques include spraying, rolling, dipping, and the like. The densification agent can be applied to one or both sides of the cellulose pulp sheet. Alternatively, the densification agent can be applied to fibers that are not in sheet form, e.g., individualized fibers. The densification agent can be heated prior to its application, although this is not required. For example, when the densification agent includes only a saccharide as described below in more detail, heating the densification agent before application and/or applying it to fibers at a temperature above about 20°C promotes the objectives of improving the densification properties of the fibers in accordance with the present invention.

As noted above, the densification agents of the present invention can include a saccharide as described above, or they can include a saccharide in combination with an agent that has at least one hydrogen bonding functionality, or they can include an agent that has at least one hydrogen bonding functionality. Useful saccharides have been described above. Suitable agents having hydrogen bonding functionality are described below in more detail.

Useful agents having at least one hydrogen bonding functionality can be either polymeric or nonpolymeric chemicals. A plurality of suitable agents having at least one hydrogen bonding functionality are described in U.S. Patent No. 5,641,561; U.S. Patent No. 5,789,326; and U.S. Patent No. 5,547,541 with reference to various polymeric binders and nonpolymeric binders. These discussions regarding the polymeric binders and nonpolymeric binders and their ability to effect the densification properties of cellulose are expressly incorporated herein by reference. Such agents have a volatility less than water. The vapor pressure of the agent may, for example, be less than 10 mm Hg at 25°C, and more preferably less than 1 mm Hg at 25°C. The agents include molecules that have at least one functional group capable of forming a hydrogen bond. Suitable densification agents include non-polymeric materials that have a functional group selected from the group consisting of a carboxyl, a carboxylate, a carbonyl, a sulfonic acid, a sulfonate, a phosphate, a phosphoric acid, a hydroxyl, an amide, an amine, and combinations thereof. As used herein, the term "non-polymeric" refers to a monomer, a dimer, a trimer, tetramer, and oligomers. Examples of agents having at least one hydrogen bonding functionality useful as a component of the densification agents of the present invention that include the functional groups set forth above include carboxylic acids, alcohols, amino acids, amino alcohols, hydroxy acids, sulfonic acids, sulfonates, amino-sulfonic acids, non-polymeric polyamides, and non-polymeric polyamines.

Suitable carboxylic acids include non-polymeric polycarboxylic acids that contain more than one carboxylic acid functional group such as citric acid, propane tricarboxylic acid, maleic acid, butane tetracarboxylic acid, cyclopentane tetracarboxylic acid, benzene tetracarboxylic acid, tartaric acid, and ascorbic acid.

Exemplary alcohols include polyols, primary alcohols, secondary alcohols, or tertiary alcohols. A polyol is an alcohol that contains a plurality of hydroxyl groups, and includes diols such as the glycols (dihydric alcohols), ethylene glycol, propylene glycol, butylene glycol, dipropylene glycol, trimethylene glycol; and triols such as glycerin. Other useful polyols include pentaerythritol and sorbitol. Esters of hydroxyl containing binders also may be used with mono- and diesters of glycerin, such as monoglycerides and diglycerides, being particular examples.

Useful amino acids include glycine, alanine, valine, serine, threonine, cysteine, glutamic acid, lysine, or beta-alanine.

Exemplary amino alcohols are alcohols that contain an amino group (-NR₂), and include ethanolamine (2-aminoethanol) and diglycolamine (2-(2-aminoethoxy) ethanol).

Useful hydroxy acids are acids that contain a hydroxyl group, including hydroxy acetic acid, lactic acid, tartaric acid, ascorbic acid, citric acid, and salicylic acid.

Useful sulfonic acids and sulfonates contain a sulfonic acid group (-SO₃H) or a sulfonate (-SO-₃).

Useful amino sulfonic acids include taurine, which is 2-aminoethane sulfonic acid.

Useful non-polymeric polyamides have more than one amide group, such as oxamide, urea, and biuret.

Useful non-polymeric polyamines include amines that have more than one amine group, such as ethylenediamine, ethylenldiaminetetraacetic acid, or the amino acids asparagine or glutamine.

Other useful agents having at least one hydrogen bonding functionality include glyoxal, phosphates and phosphoric acids.

Each of the agents described above is capable of forming hydrogen bonds because it has a functional group that contains electronegative atoms, particularly oxygens or nitrogens, or has electronegative groups, particularly groups containing oxygens or nitrogens and that also includes a hydrogen. The amino alcohols, amino acids, carboxylic acids, alcohols and hydroxy acids all have a hydroxyl group in which a hydrogen is bound to an electronegative oxygen, creating a dipole that leaves the hydrogen partially positively charged. The amino alcohols, amino acids, amides, and amines all have an NR group in which a hydrogen may be bound to an electronegative nitrogen that also leaves the hydrogen partially positively charged. The partially positively charged hydrogen in both cases can interact with an electronegative element, such as oxygen or nitrogen, to form hydrogen bonds. The polycarboxylic acids, hydroxy acids, amino acids, and amines also have a carboxyl group with an electronegative oxygen that can interact with hydrogen atoms. Similarly, hydrogen atoms that have positive dipoles can interact with electronegative atoms such as oxygen or nitrogen to form hydrogen bonds.

Particular families of agents having hydrogen bonding functionality useful as a component in the densification agents of the present invention include alcohols, hydroxy acids, and polycarboxylic acids. These families of agents having hydrogen bonding functionality are desirable due to their general acceptance by customers of the products into which the fibers of the present invention are incorporated.

The following discussion proceeds with reference to these specific examples of agents having hydrogen bonding functionality, however, it should be understood that the present invention is not so limited. The following description also discusses the present invention with reference to the specific saccharide high fructose corn syrup; however, it should be understood that the present invention is not so limited. The following description is equally applicable to a densification agent that includes only a saccharide or one that includes only an agent having at least one hydrogen bonding functionality.

As noted above, a densification agent that includes a saccharide and at least one material having hydrogen bonding functionality can be applied to cellulose fibers that have been treated with an oil to produce fibers that exhibit desirable densification properties. The densification agents can be formed by mixing saccharide(s) with the agent(s) having at least one hydrogen bonding functionality. Water may be added to the mixture depending on the water content of the various components.

In particular embodiments, the weight ratio of the saccharide to the agent(s) having at least one hydrogen bonding functionality in the densification agent ranges from 100:0, that is, saccharide only to about 0:100, that is, no saccharide present, only the non-saccharide densification agent. A narrower range for the ratio of saccharide to non-saccharide actives component in the densification agent ranges from about 70:30 to about 85:15. An exemplary ratio is 80:20 saccharide : other agent. The foregoing weight ratios are based on the weight of the actives in the components making up the densification agent. It should be understood that as the amount of the densification agent applied varies, the amount of the various components in the densification agent can change in order to achieve the desired levels of agent loading described above.

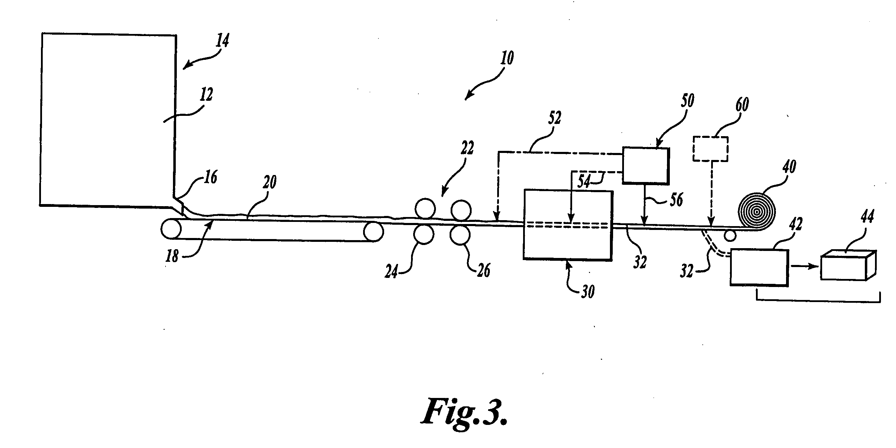

FIGURE 3 illustrates a wet laid sheet manufacturing line such as a wood cellulose pulp sheet manufacturing line 10. In this manufacturing line, a pulp slurry 12 is delivered from a headbox 14 through a slice 16 and onto a Fourdrinier wire 18. The pulp slurry 12 typically includes wood pulp fibers and may also include synthetic or other non-cellulose fibers as part of the slurry. Water is drawn from the pulp deposited on wire 18 by a conventional vacuum system, not shown, leaving a deposited pulp sheet 20 which is carried through a dewatering station 22, illustrated in this case as two sets of calendar rolls 24, 26 each defining a respective nip through which the pulp sheet or mat 20 passes. From the dewatering station, the pulp sheet 20 enters a drying section 30. In a conventional pulp sheet manufacturing line, drying section 30 may include multiple canister dryers with the pulp mat 20 following a serpentine path around the respective canister dryers and emerging as a dried sheet or mat 32 from the outlet of the drying section 30. Other alternate drying mechanisms, alone or in addition to canister dryers, may be included in the drying stage 30. The dried pulp sheet 32 has a maximum moisture content pursuant to the manufacturer's specifications. Typically, the maximum moisture content is no more than 10% by weight of the fibers and most preferably no more than about 6% to 8% by weight. Unless overly damp fibers are immediately used these fibers are subject to degradation by, for example, mold or the like. The dried sheet 32 is taken up on a roll 40 for transportation to a remote location, that is, one separate from the pulp sheet manufacturing line, such as at a user's plant for use in manufacturing products. The dried pulp sheets have a basis weight of about 200 g/m² to about 1000 g/m² or more and a density on the order of at least about 0.5 g/cm³ to about 1.2 g/cm³. Dried pulp sheets having the foregoing basis weights are structurally distinct form lighter basis weight sheets of wet laid or airlaid wood pulp fibers such as tissue paper, paper towels, or other types of paper-like wet laid or airlaid webs of cellulose fibers. Alternatively, the dried sheet 32 is collected in a baling apparatus 42 from which bales of the pulp 44 are obtained for transport to a remote location.

The oil and densification agents of the present invention can be applied to the pulp sheet from one or more applying devices, one of which is indicated at 50 in FIGURE 3. Any applying device may be used, such as streamers, sprayers, roll coaters, curtain coaters, immersion applicators, or the like. Sprayers are typically easier to utilize and incorporate into a pulp-sheet manufacturing line. As indicated by the arrows 52, 54, and 56, the oil and densification agents may be applied at various locations or at multiple locations on the pulp sheet manufacturing line, such as ahead of the drying stage 30 (indicated by line 52), intermediate the drying stage 30 (as indicated by line 54), or downstream from the drying stage 30 (as indicated by the line 56). At location 52, the water remaining in the sheet or mat 20 at this stage tends to interfere with the penetration of the materials into the sheet. Consequently, application of the oil and densification agent after some drying has taken place, for example at location 54, is preferable. If the oil and densification agent is applied at location 56 in an amount which would cause the moisture content of the sheet to exceed the desired maximum level, an additional drying stage (not shown) may be included in the pulp manufacturing line to bring the moisture content down to the desired level.

The rolls 40 or bales 44 of the treated wet laid web of fibers may be transported to a remote location for use by a user. These rolls or bales are then refiberized by a fiberizing device, such as a hammermill which may be used alone or in conjunction with other devices such as picker rolls or the like for breaking up the sheet 32 or bales 42 into individual fibers. Depending on the end use, the individualized fibers may be combined with particulate material, such as superabsorbent particles, and/or airlaid into a web and densified.

With this approach, the end user of the treated fibers may readily select particles to be combined with the fibers. The user has flexibility in air laying or otherwise processing the treated fibers of the present invention into a finished product.

The oil and densification agent treated fibers and superabsorbent material can be combined and then formed into an absorbent structure in the following manner. Rolls or bales of treated fibers, without particles, are fiberized by a fiberizing device such as a hammermill. The individualized fibers are air entrained during which time the superabsorbent material can be added thereto. The air entrained fibers and superabsorbent material are then delivered to an air laying device, such as a pocket former, and formed into a desired shape. The formed pad is removed from the air laying device for further processing, including subjecting the pad to a compression load to reduce the thickness of the pad and increase its density. The formed pads are in the form of a web or mass of fibers used as absorbent structures in absorbent articles such as the ones discussed above. The webs or masses of fibers have basis weights ranging from about 100 to about 1000 grams per square meter (gsm), thicknesses ranging from about 1-6.66 millimeters and densities ranging from 0.15 to about 1 g/cm³.

It should be understood that in an alternative embodiment, the oil and densification agent can be applied to the fibers while they are air entrained.

As illustrated in the examples that follow, the addition of densification agents to oil treated fibers in accordance with the present invention does not significantly affect the superabsorbent retention properties of the oil treated fibers. As illustrated in the examples that follow, oil treated fibers treated with a densification agent in accordance with the present invention exhibit desirable densification properties.

The following examples are intended to illustrate certain embodiments of the present invention and are not intended to limit the scope of the present invention.

### Example 1

### Preparation of Oil and High Fructose Corn Syrup Treated Cellulose Pulp

Two 100-foot rolls of Southern Pine fluff in sheet form available from Weyerhaeuser Company under the designation NB 416 from New Bern, North Carolina with a starting moisture content of 6% by weight (based on total sheet weight) were coated in a Black Brothers gravure-type roll coater with a solution of high fructose corn syrup. The gravure coater results in the application of a uniform coating of the high fructose corn syrup solution over one entire surface of the pulp sheet from where it is rapidly soaked up by the sheet. The high fructose corn syrup was obtained from Archer-Daniels Midland Company of Decatur, Illinois under the trademark CORN SWEET® 42. The high fructose corn syrup had an actives content of 71% with the balance being water. The high fructose corn syrup was diluted with water to an active content of 48.6 wt. % based on total solution weight and applied to the wood pulp sheet to achieve a loading of actives on a dry basis (i.e., corn syrup solids) of 5 wt. % based on the dry fiber content of the pulp sheet.

After application of the high fructose corn syrup, the opposite side of the sheets were coated with an oil using a Black Brothers gravure-type roll coater. The oil was a mineral oil obtained from Chevron Texaco Corporation under the designation Superla 35. The mineral oil was applied to a loading of 3 wt. % oil based on the dry fiber content of the sheet.

The resulting sheets were fiberized and formed into pads using a Fitz hammermill feeding an M&J continuous air lay pad forming device. Pads were formed comprising treated fibers only, having a basis weight of 300 grams per square meter, and pads comprising 60 wt. % treated fibers and 40 wt. % superabsorbent material for a total basis weight of 500 grams per square meter.

Three 10cm x 10cm pads were cut from the larger formed pads and conditioned for at least two hours at 50% relative humidity. Each pad was then subjected to a compression load sequentially of 50 psi, 100 psi, and 150 psi, using a platen press. The pressure applied by the platen press was relieved immediately upon reaching the target pressure value. The density of each pad at each pressure increment was measured within 5 seconds of relieving the pressure. The density of the pad was determined by measuring pad thickness by using a device that does not materially compress the sample, weighing the pad, and calculating density as Density = Weight/(10cm x 10cm x Thickness), where the units of thickness are cm, weight is grams, and density is grams/cubic centimeter. The results are expressed as Sample 2 in Table 1 below as a percentage of the density achieved when a control comprising untreated wood pulp fiber was processed as described above.

The foregoing procedure was repeated with the exception that the oil loading was 5 wt. % on dry fiber. The results for this sample are summarized in Table 1 below under the designation Sample 3.

The above procedure for Sample 3 was repeated, substituting an 80/20 wt. to weight percent blend of actives from the high fructose corn syrup and propylene glycol. The propylene glycol was obtained from Integra Chemical of Renton, Washington, and had a water content of less than one percent. The resulting high fructose corn syrup and propylene glycol solution was 52.7 wt. % actives, the balance water.. This composition was applied to the pulp sheets to a loading of 6 wt. % actives based on the weight of dry fibers. The results for this sample are summarized in Table 1 below under Sample 4.

The procedure above for Sample 2 was repeated on a single 100-foot roll of pulp. For this sample, no high fructose corn syrup or propylene glycol was applied and only mineral oil was applied to a loading of 10.6 wt. % on dry fiber. When this pulp sample was fiberized, it was fed to the hammermill device along with a roll of untreated pulp. This combination of two sheets feeding the hammermill, one coated and the other not, lead to a final oil loading of 5.3 wt. % on dry fiber. The results for this sample are summarized in Table 1 below under Sample 5 as a comparative example.

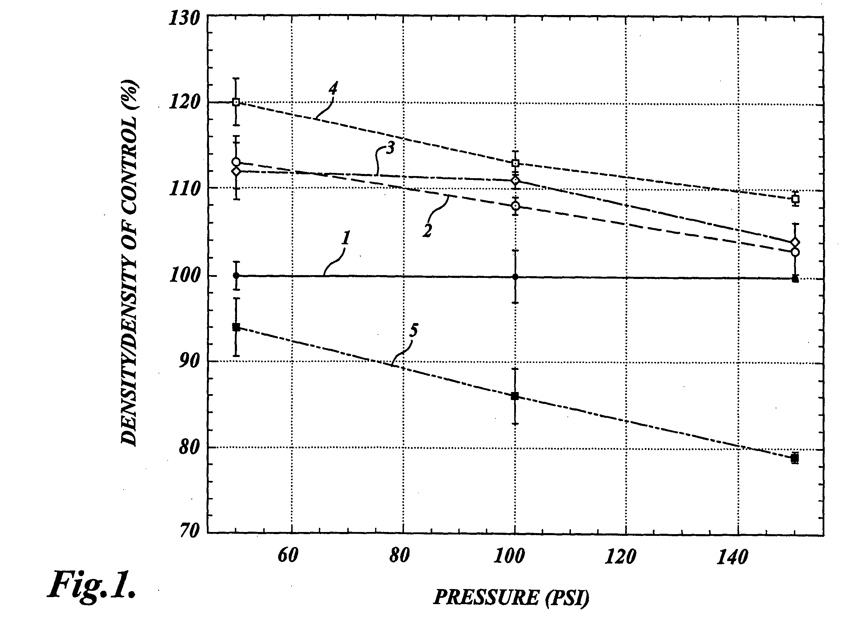

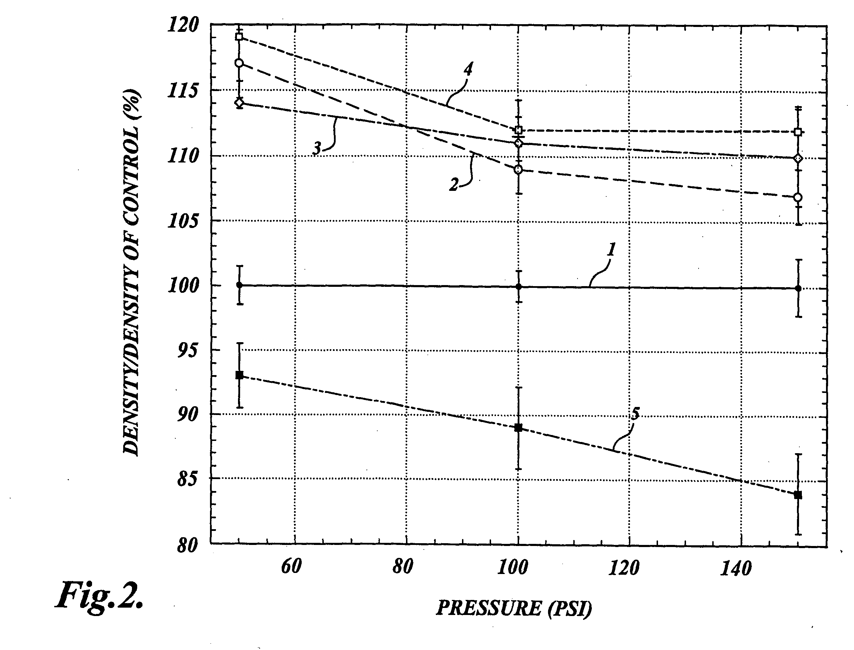

The information set forth in Table 1 is graphically illustrated in FIGURE 1 (no sap) and FIGURE 2 (40 wt. % superabsorbent material). The reference numbers in FIGURES 1 and 2 correspond to the Sample Nos. in Table 1.

The examples above illustrate that the application of high fructose corn syrup alone or in combination with an agent having at least one hydrogen bonding functionality to wood pulp cellulose fibers treated with an oil improves the densification properties of the oil-treated fibers.

**TABLE 1**

| Density of Sample/Density of Control Expressed as a Percent | | | | | | |
|---|---|---|---|---|---|---|
| | FIBERS ONLY | | | FIBER AND SAP | | |
| Pressure Load (psi) | 50 | 100 | 150 | 50 | 100 | 150 |
| Sample 1 (control) | 100 | 100 | 100 | 100 | 100 | 100 |
| Sample 2 | 113.1 | 108.4 | 102.8 | 116.8 | 109.3 | 107.0 |
| Sample 3 | 112.3 | 110.6 | 103.9 | 114.3 | 110.6 | 109.7 |
| Sample 4 | 119.9 | 112.9 | 109.2 | 118.8 | 112.4 | 112.3 |
| Sample 5 (Comparative Example) | 94 | 86 | 79 | 93 | 89 | 84 |

## Claims

1. A cellulose pulp sheet comprising:
cellulose fibers;
an oil applied to the cellulose fibers; and
a densification agent applied to the cellulose fibers.

2. The sheet of Claim 1 wherein the cellulose fibers are wood pulp fibers.

3. The sheet of Claim 1 or 2 wherein the oil has a melting point below 25°C.

4. The sheet of any one of Claims 1 to 3 wherein the oil comprises a triglyceride.

5. The sheet of any one of Claims 1 to 3 wherein the oil is a fatty acid.

6. The sheet of any one of Claims 1 to 3 wherein the oil is olive oil, soybean oil, safflower oil, cottonseed oil, linseed oil, tung oil, castor oil, coconut oil, canola oil, corn oil, or jojoba oil.

7. The sheet of any one of Claims 1 to 3 wherein the oil is saturated or unsaturated alkane, alkene, alkyne, cycloalkane, cycloalkene, cycloalkyne, or combinations thereof.

8. The sheet of any one of Claims 1 to 3 wherein the oil is petroleum derived.

9. The sheet of Claim 8 wherein the oil is mineral oil, hexadecane, squalane or squalene.

10. The sheet of any one of the preceding Claims wherein the oil is present on the fibers in an amount ranging from 0.5 to 20 wt.% based on the weight of dry fibers.

11. The sheet of any one of the preceding Claims wherein the densification agent comprises a saccharide.

12. The sheet of claim 11 wherein the saccharide is a high fructose corn syrup.

13. The sheet of any one of the preceding Claims wherein the densification agent comprises an agent having at least one hydrogen bonding functionality.

14. The sheet of Claim 11 or 12 wherein the densification agent further comprises at least one agent having hydrogen bonding functionality.

15. The sheet of Claim 13 or 14 wherein the agent having at least one hydrogen bonding functionality is an alcohol, hydroxy acid or polycarboxylic acid.

16. The sheet of Claim 15 wherein the alcohol is a polyol or glycol.

17. The sheet of Claim 15 wherein hydroxy acid is lactic acid.

18. The sheet of Claim 13 or 14 wherein the agent having at least one hydrogen bonding functionality is propylene glycol.

19. The sheet of any one Claims 13 to 18 wherein the weight ratio of saccharide actives to actives in the at least one agent having a hydrogen bonding functionality in the densification agent ranges from 70:30 to 85:15.

20. The sheet of any one of the preceding Claims wherein the densification agent is present in an amount ranging from 0.5 to 20 wt.% actives based on dry weight of the fibers.

21. The sheet of any one of the preceding Claims having a densification agent actives content ranging from 3 to 8 wt.%.

22. A method for producing a cellulose pulp sheet comprising:
providing cellulose pulp;
forming a cellulose pulp sheet from the cellulose pulp;
applying an oil to the cellulose pulp sheet; and
applying a densification agent to the cellulose pulp sheet.

23. The method of Claim 22 wherein the oil and the densification agent are applied to the cellulose pulp sheet simultaneously.

24. The method of Claim 22 wherein the oil is applied to a first side of the cellulose pulp sheet and the densification agent is applied to a second side of the cellulose pulp sheet, the first side being opposite the second side.

25. A method for producing a densified web of cellulose fibers comprising:
providing cellulose fibers treated with an oil and a densification agent;
fiberizing the cellulose fibers treated with the oil and the densification agent;
forming the fiberized cellulose fibers treated with the oil and the densification agent into a web; and
compressing the web.

26. A method for modifying the densification properties of cellulose fibers treated with an oil, the cellulose fibers before treatment with the oil and after application and removal of a compression load being densified to a first density, the cellulose fibers after treatment with the oil and after application and removal of the compression load being densified to a second density, the first density being greater than the second density, the method comprising:
applying a densification agent to the oil treated fibers, the densification agent applied to the oil treated fibers in an amount that results in the fibers treated with the oil and the densification agent densifying to a third density after application and removal of the compression load, the third density being greater than the first density.

27. The method Claim 26 wherein the third density is at least 2% greater than the first density when the compression load is 150 psi or less.

28. The method of any one of Claims 22 to 27 wherein the densification agent comprises an agent having at least one hydrogen bonding functionality.

29. The method of any one of Claims 22 to 28 wherein the densification agent comprises a saccharide.

30. The method of Claim 29 wherein the densification agent further comprises an agent having at least one hydrogen bonding functionality.

31. The method of any one of Claims 22 to 30 wherein the densification agent is applied to the cellulose fibers in an amount resulting in an application of 0.5 to 20 wt% actives based on dry weight of the cellulose fibers.

32. An article for absorbing an aqueous fluid, comprising:
cellulose fibres;
superabsorbent materials; and
an oil and a densification agent applied to the cellulose fibers.

33. The article of Claim 32 wherein the densification agent comprises an agent having at least one hydrogen bonding functionality.

34. The article of Claim 32 or 33 wherein the densification agent comprises a saccharide.

35. The article of Claim 34 wherein the densification further comprises an agent having at least one hydrogen bonding functionality.

36. The article of any one of Claims 32 to 35 wherein the densification agent is present in an amount such that 0.5 to 20 wt.% actives based on dry weight of the cellulose fibers is present on the cellulose fibers.
